# EUROPEAN PATENT SPECIFICATION

(11) **EP 0 806 963 B1**
(45) Date of publication and mention of the grant of the patent: **01.08.2001**
(21) Application number: 95938518.8
(22) Date of filing: 01.12.1995
(51) Int. Cl.: A61K 39/00, A61K 39/395, A61K 38/18, C07K 14/475

(54) **CARCINOMA TREATMENT**
KARZINOMBEHANDLUNG
TRAITEMENT DES CARCINOMES

(30) Priority: 13.12.1994 GB 9425060
(43) Date of publication of application: 19.11.1997
(73) Proprietor: THE UNIVERSITY OF BIRMINGHAM, Edgbaston, Birmingham B15 9TB (GB)
(72) Inventor: YOUNG, Lawrence, Sterling, West Midlands B62 8SP (GB)
(74) Representative: Pearce, Anthony Richmond
(86) International application number: GB9502807
(87) International publication number: WO9618413

(56) References cited:
- EP-A- 0 585 943
- BLOOD, vol. 86 (10 SUPPL. 1), 1995 page 318A, Document 1261 M. A. DEGLI-ESPOSTI ET AL. 'CD40 ligand, a potent growth factor, causes apoptosis of bladder carcinoma cell lines.'
- IMMUNOLOGY, vol. 79, no. 3, 1993 pages 445-451, L. FLORES-ROMO ET AL. 'Anti-CD40 antibody stimulates the VLA-4-dependent adhesion of normal and LFA-1-deficient B cells to endothelium'
- NATURE, vol. 357, no. 6373, 1992 pages 80-82, RICHARD J. ARMITAGE ET AL. 'Molecular and biological characterization of a murine ligand for CD40'
- INT. J. CANCER, vol. 43, 1989 pages 786-794, L. S. YOUNG ET AL. 'Identification of a human epithelial cell surface protein sharing an epitope with the C3d/Epstein-Barr virus receptor molecule of B lymphocytes'

## Description

This invention relates mainly to carcinoma treatment and is more particularly concerned with the treatment of carcinomas in epithelial cell systems, although the present invention, in its broadest aspect, relates generally to the prevention of epithelial cell proliferation.

It is known to treat carcinomas in epithelial cell systems using anti-neoplastic agents which are intended to induce apoptosis. Examples of such agents include cytokines and growth factors such as transforming growth factor β₁ (TGF-β₁), anti-Fas, and tumour necrosis factor-α (TNF-α) and other antineoplastic agents such as interferon-γ (IFN-γ), cis-platin, mitomycin C, cyclophosphamide, actinomycin D, doxorubicin, vincristine, etoposide, 5-fluorouracil, methotrexate and irradiation agents. However, there is a continuing need for other drugs for treatment of carcinomas in epithelial cell systems and/or for enhancing the effect of existing anti-neoplastic agents.

EP-A-0585943 discloses the use of soluble gp39 (a CD40 receptor ligand) together with co-stimulating agents, to promote the proliferation of B-cells, where for example, such proliferation may be desirable in the treatment of conditions benefitting from an augmented immune response.

In a first aspect, the present invention resides in the use of a CD40 receptor binder for the manufacture of a medicament for the prevention of epithelial cell proliferation or for the treatment of carcinomas in epithelial cell systems.

In a second aspect, the present invention resides in the use of a CD40 receptor binder in the manufacture of a medicament for enhancing the susceptibility of neoplastic epithelial cells to anti-neoplastic drug-induced apoptosis.

In a third aspect, the present invention resides in the use of a CD40 receptor binder in combination with a CD40 inducer for the prevention of epithelial cell proliferation or for the treatment of carcinomas in epithelial cell systems.

In a fourth aspect, the present invention resides in the use of a CD40 receptor binder in combination with an anti-neoplastic drug for the treatment of carcinomas in epithelial cell systems.

In a fifth aspect, the present invention resides in a method of preventing epithelial cell proliferation, comprising administering a CD40 receptor binder to a human or animal patient.

In a sixth aspect, the present invention resides in a method of treating an epithelial cell carcinoma in a human or animal patient, comprising administering a CD40 receptor binder to the patient.

Preferably, such administration is effected in conjunction with administration of at least one anti-neoplastic agent and/or a CD40 inducer.

The anti-neoplastic agent may be selected from known cytokines and growth factors such as transforming growth factor β₁ (TGF-β₁), anti-Fas, and tumour necrosis factor-α (TNF-α) and other known antineoplastic agents such as interferon-γ (IFN-γ), *cis*-platin, mitomycin C, cyclophosphamide, actinomycin D, doxorubicin, vincristine, etoposide, 5-fluorouracil, methotrexate and irradiation agents. Such anti-neoplastic agent will normally be administered at a dose rate consistent with the recommended for that particular agent.

The CD40 inducer may be interferon-γ (IFN-γ), tumour necrosis factor-α (TNF-α) or any other known inducer of CD40 expresssion. Such CD40 inducer will normally be used at a dose rate consistent with that recommended for that particular inducer.

CD40 is a 50-kDa type 1 glycoprotein belonging to the nerve growth factor receptor/tumour necrosis factor receptor (NGF-R/TNF-R) family, and is expressed on the surface of B lymphocytes (see Noelle et al., Immunol. Today, **13**, 431-433, 1992; and Smith et al., Cell, **76**, 959-962, 1994). For both normal and neoplastic B cells prone to enter or be driven into apoptosis, ligation of CD40 provides a potent rescue (survival) signal which is partially mediated through the up-regulation of Bcl-2 expression (see Liu et al., Nature, **342**, 929-931, 1989). This effect was shown initially using ligand-mimetic monoclonal antibodies (mAb) and more recently confirmed with recombinant ligand (CD40L) (see Noelle et al., **supra).**

It is also known that expression of both CD40 and Bcl-2 occurs in normal, basal epithelial cells and in a number of different carcinomas including those of the ovary, nasopharynx, lung and breast (see Young et al., Int. J. Cancer, **43**, 786-794, 1989; and Hockenbery et al., Proc. Natl. Acad. Sci. USA, **88**, 6961-6965, 1991). These studies suggest that the CD40 pathway may be active in carcinomas and that the resulting expression of Bcl-2 may protect these tumours from apoptotic cell death including that induced by the cytotoxic drugs used in conventional cancer chemotherapy (see Dive et al., Semin. Cancer Biol, **3**, 417-427, 1992). Furthermore, the restricted expression of CD40 and Bcl-2 to the proliferative basal compartment of stratified, squamous epithelium indicates a possible role for this pathway in regulating normal growth and the apoptotic process of epithelial differentiation.

In view of the above, it was surprising to find that, contrary to the studies in B cells, CD40 stimulation in epithelial cells of either normal or neoplastic origin results in growth arrest and enhanced susceptibility to anti-neoplastic agent-induced apoptosis.

As examples of CD40 binders, there may be used ligand-mimetic monoclonal antibodies, for example G28.5 (a hybridoma available under the Accession No. HB-9110 from the American Type Tissue Culture Collection, Rockville, Maryland, USA), S2C6 (as disclosed by Paulie et al, Cancer Immunology and Immunotherapy, 20, 23-28, 1985), and naturally occuring or engineered forms of CD40 ligands, of which an example is CD40L. CD40L is a 39-kDa type II integral membrane protein with homology to TNF, which can be induced on T cells following their activation via the T cell receptor (see Clark & Ledbetter, Nature, **367**, 425-428, 1994). The CD40-CD40L pair are part of an expanding family of interacting receptor-ligand molecules based on the TNF-R/TNF families which are involved in cellular activation and regulation of apoptosis in target cells (Smith et al., **supra**).

The CD40 binders may be administered by intravenous, subcutaneous or intraperitoneal injection or infusion, or by direct injection into the site of the tumour in purified or humanised form. The dose rate for the CD40 depending upon whether it is a monoclonal antibody or CD40L and may lie in the microgram to milligram range.

Pharmaceutically acceptable carriers (diluents, excipients, etc.) which are used with the CD40 binder can be selected from any of the known carriers for these types of drug and will depend, inter alai, upon the mode of administration. For example, for producing an injectable composition, the carrier may be selected from simple preparations such as phosphate-buffered saline containing human serum albumin to more complex solutions such as cell culture media.

The present invention will now be described in further detail in the following examples and with reference to the accompanying figures, in which:-
Fig 1 is a chart showing the effect of 48 hours treatment with anti-CD40 mAb G28.5 on the growth of ovarian carcinoma cell line A2780, the data representing the mean of triplicate determinations from three independent experiments and being presented as % decrease in survival relative to untreated controls,
Fig 2 is a chart showing that pre-incubation of 2780CP cell line with 150 U/ml IFN-γ for 24 hours induces a 26% decrease in cell growth in the presence of 2 *µ*g/ml G28.5 mAb,
Fig 3 is a chart showing the effect of time and concentration of G28.5 mAb on the cell growth of an EJ bladder carcinoma cell line,
Fig 4 is a chart showing the effect of anti-CD40 mAb on the growth of Rat-1/CD40 transfectant (CI.8),
Fig 5 is a chart showing the enhancement of cytotoxicity by the combination of G28.5 mAb and 5 *µ*M *cis*-platin in the ovarian carcinoma cell line A2780, cytotoxicity being measured in a 48 hour MTT assay, the data representing the mean of triplicate determinations from three independent experiments and being presented as % decrease in survival relative to untreated controls,
Fig 6 is a chart showing the enhancement of cis-platin-induced cytotoxicity in an EJ bladder carcinoma cell line by incubation for 48 hours with 2 *µ*g/ml G28.5 mAb,
Fig 7 is a chart showing the effect of the incubation of Rat-1/CD40 CI.8 cells with 1*µ*g/ml G28.5 mAb on various concentrations of *cis*-platin, the data representing the mean of triplicate determinations from three independent experiments,
Fig 8 is a chart showing the effect of 48 hours treatment with CD40L on the growth of the ovarian carcinoma cell line A2780, the data representing the mean of triplicate determinations from three independent experiments and being presented as % decrease in survival relative to untreated controls,
Fig 9 is a chart showing the effect of time and concentration of CD40L on the cell growth of an EJ bladder adenocarcinoma cell line,
Fig 10 is a chart showing the effect of CD40L on the growth of a Rat-1/CD40 transfectant (CI.8), and
Fig 11 is a chart showing the enhancement of cis-platin-induced cytotoxicity in the EJ bladder carcinoma cell line by incubation for 48 hours with 100 U/ml CD40L.

### Example 1

### Cell culture

A human ovarian carcinoma cell line A2780 and its cis-platin-resistant derivative 2780CP ( see Masuda et al., Cancer Res., **50**, 1863-1866, 1990), an EJ bladder carcinoma cell line (Accession No.85061108, European Collection of Animal Cell Cultures, Porton Down, UK ) and Rat-1 fibroblasts (Lania et al., Virology, **101,** 217-232, 1980) were continuously maintained in RPMI 1640 medium (GIBCOBRL, Life technologies Ltd, Paisley, Scotland) supplemented with 10% FCS (foetal calf serum - ICN Flow, Thane, Oxfordshire, England), 2mM glutamine, and the antibiotics, penicillin (1000 U/ml) and streptomycin (1mg/ml) (Sigma Co. Ltd, Poole, Dorset, England) at 37°C in a 5% CO₂ atmosphere.

Rat-1 fibroblasts expressing CD40 were generated by electroporation of the CD40 expression vector (following the procedure described by Stamenkovic et al., EMBO J., 8, 1403-1410, 1989) with a suitable drug resistant plasmid, pUC LTR neo, at a ratio of 10:1. Stable transfectants of Rat 1 cells expressing CD40 were isolated after prolonged exposure to Geneticin G418 antibiotic at which time individual colonies of drug-resistant cells were harvested and separately cultured as individual colonies. Samples of such transfectants have been deposited under the provisions of the Budapest Treaty with European Collection of Cell Cultures, Centre for Applied Microbiology and Research, Salisbury, Wiltshire SP4 0JG, Great Britain, on 10 November 1995 under the Accession No. 951110100.

Expression of the CD40 in the above cell lines was confirmed with FACS analysis using the anti-CD40 antibody, G28.5 mAb. Briefly, cells were harvested after trypsinisation, washed in phosphate-buffered saline (PBS) and resuspended in 0.5 *µ*g/ml G28.5 mAb. After 1 hour incubation at 4°C, the cells were washed in PBS and resuspended in 1:50 dilution of FITC-conjugated goat anti-mouse IgG (Sigma Chemical Co. Ltd, Poole, Dorset, England). After a further 30 minute incubation at 4°C, the cells were washed and finally resuspended in 1% paraformaldehyde prior to standard FACS analysis.

### Treatment of cells and Assay for Cell Growth

Inhibition of cell proliferation was evaluated using the colorimetric assay of Mossman (see Mossman, J. Immunol. Meths., 65, 55-63, 1983). In brief, cells were plated on a 96 well plate overnight to enter normal cell cycle. Various concentrations of G28.5 mAb were then added and their effect on cell growth was evaluated after an appropriate period of time (48, 72 or 96 hrs) by the addition of 20 *µ*l of 5 mg/ml MTT (3-(4,4-dimethylthiazol-2-yl)-2,5-diphenyl tetrazolium bromide) obtained from Sigma, in PBS. The cells were further incubated for 5 hrs at 37°C, formazan crystals thus formed were dissolved in DMSO and the optical density (O.D.) was recorded at 550nm on a Becton Dickinson Multiscan. In a further series of experiments, cells were treated for 2 hrs with various *cis*-platin concentrations with or without G28.5 mAb. The cells were then washed twice in PBS and 200*µ*l of fresh medium containing G28.5 mAb. After 48 hours, cell growth was assessed using the MTT assay as described above.

### Effect of anti-CD40 mAb on carcinoma cell growth

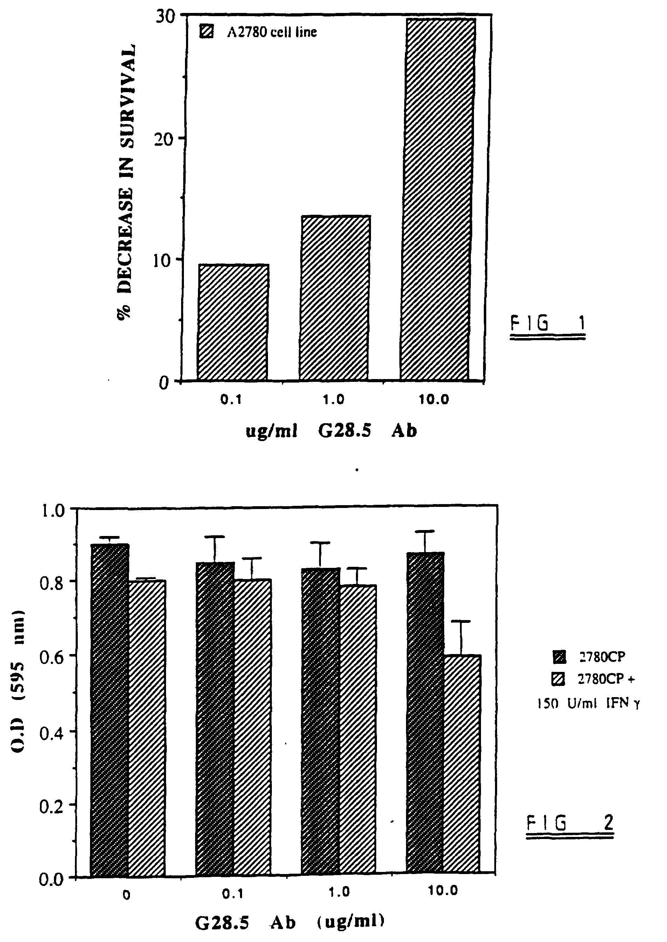

The effect of the anti-CD40 mAb G28.5 on the cell growth of the A2780, 2780CP and EJ carcinoma cell lines as well as a CD40 Rat-1 transfectant (Rat-1/CD40 CI.8) was examined. G28.5 mAb at the optimum concentration of 10 *µ*g/ml induced a 29% decrease in survival of the ovarian A2780 cell line relative to control after 48 hrs treatment (Fig.1).

When the *cis*-platin-resistant 2780CP cell line was treated under similar conditions with G28.5 mAb, no effect was observed. This was probably due to the low level of CD40 expression in this cell line. However, treatment of the 2780CP cell line with 150 U/ml of interferon-γ(IFNγ) for 24 hrs and subsequent incubation with 10 *µ*g/ml G28.5, induced an approximately 22% decrease in survival consistent with the induction of CD40 by IFNγ (Fig. 2). Similar effects of CD40 activation were observed with the EJ bladder carcinoma cell line (Fig. 3). The effect of G28.5 mAb on the EJ cell line is both time- and concentration-dependent with maximum effect at 96 hrs where treatment with 2 *µ*g/ml G28.5 induced a *27%* decrease in survival (Fig. 3).

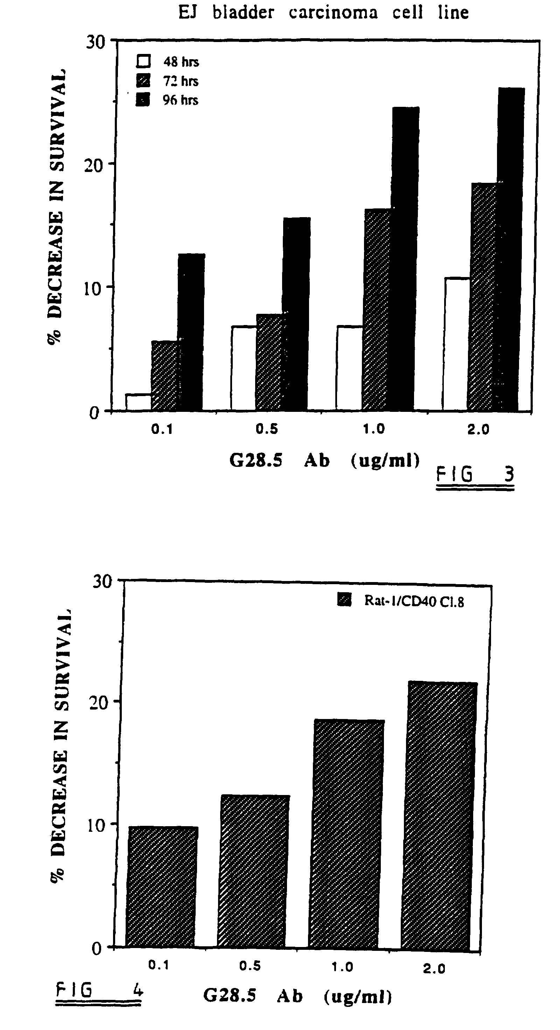

The specificity of the CD40 effect was investigated using Rat-1 fibroblasts expressing a transfected CD40. As demonstrated in Fig. 4, stimulation of CD40 by the G28.4 mAb inhibited cell growth. In the transfectant studied (clone 8), incubation for 48 hrs with 2 *µ*g/ml G28.5 mAb induce a 22% decrease in cell growth (Fig. 4).

### Enhancement of cis-platin-induced cell death by CD40 activation

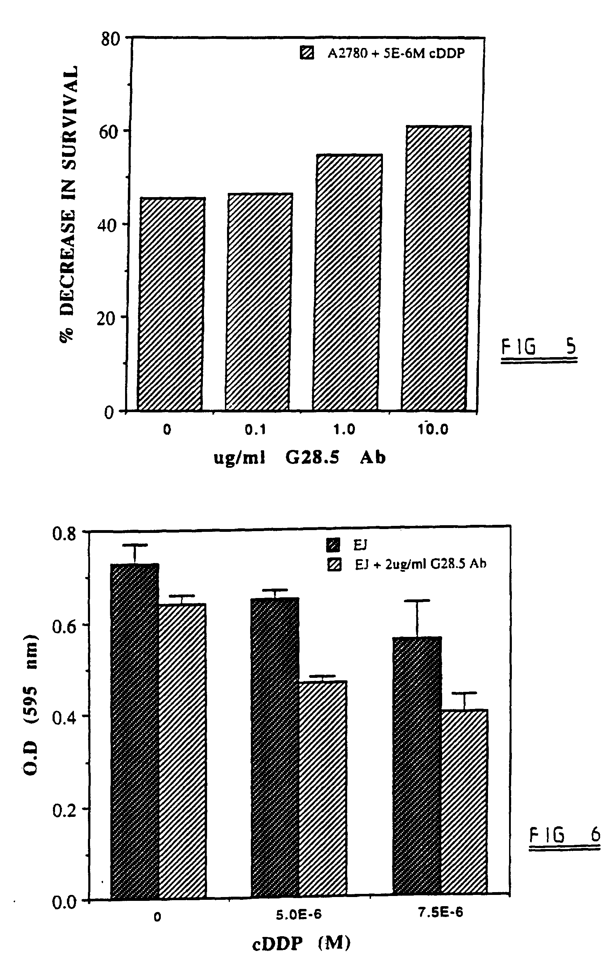

Treatment of the above cell lines with G28.5 mAb enhanced the cytotoxic effect of anti-cancer drugs such as *cis*-platin. In the A2780 cell line, exposure to 10 *µ*g/ml G28.5 mAb increased the cell killing effect of 5 *µ*M *cis*-platin from 46% to 61% (Fig. 5), which represents a statistically significant effect. Similar results were obtained with the EJ line treated with 5 *µ*M *cis*-platin in the presence of 2 *µ*g/ml G28.5 mAb (Fig. 6).

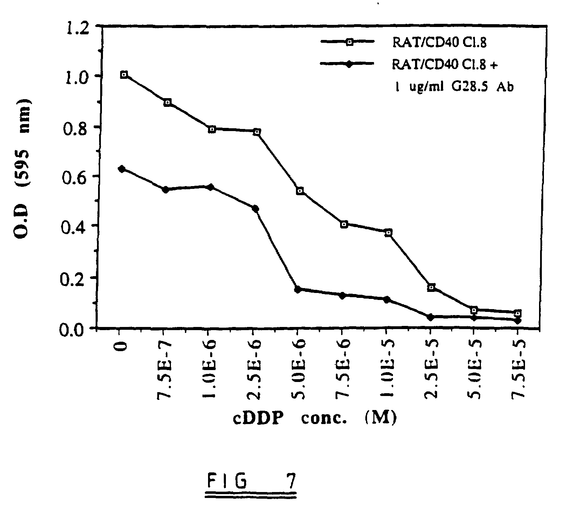

Treatment of the Rat-1/CD40 CI.8 cell line with 1 *µ*g/ml G28.5 mAb also enhanced the cytotoxic effect of *cis*-platin by shifting the IC50 value from 6 *µ*M (+/- 0.9) to 4.2 *µ*M (+/- 0.3), representing a 1.6-1.8 fold increase in drug sensitivity (Fig 7). No effect was found in parental untransfected Rat-1 cells).

### Example 2

### Cell culture

The cell culture techniques described hereinabove in Example 1 were utilised.

### Treatment of cells and Assay for Cell Growth

Inhibition of cell proliferation was evaluated using the colorimetric assay of Mossman (Mossman, J. Immunol. Meths. 65, 55-63, 1983). In brief, cells were plated on a 96 well plate overnight to enter normal cell cycle. Various concentrations of CD40L (an engineered soluble form of a CD40 binding ligand (Immunex Research and Development Corporation, 51 University Street, Seattle, Washington, USA) were then added and their effect on cell growth was evaluated after the appropriate period of time (48, 72 or 96 hrs) by the addition of 20 *µ*l of 5 mg/ml MTT (3-(4,4-dimethylthiazol-2-yl)-2,5-diphenyl tetrazolium bromide) obtained from Sigma, in PBS. The cells were further incubated for 5 hrs at 37°C, formazan crystals thus formed were dissolved in DMSO and the optical density (O.D.) was recorded at 550nm on a Becton Dickinson Multiscan. In another series of experiments, cells were treated for 2 hrs with various *cis*-platin concentrations with or without CD40L. The cells were then washed twice in PBS and 200*µ*l of fresh medium containing CD40L. After 48 hours, cell growth was assessed using the MTT assay as described above.

### Effect of CD40L on carcinoma cell growth

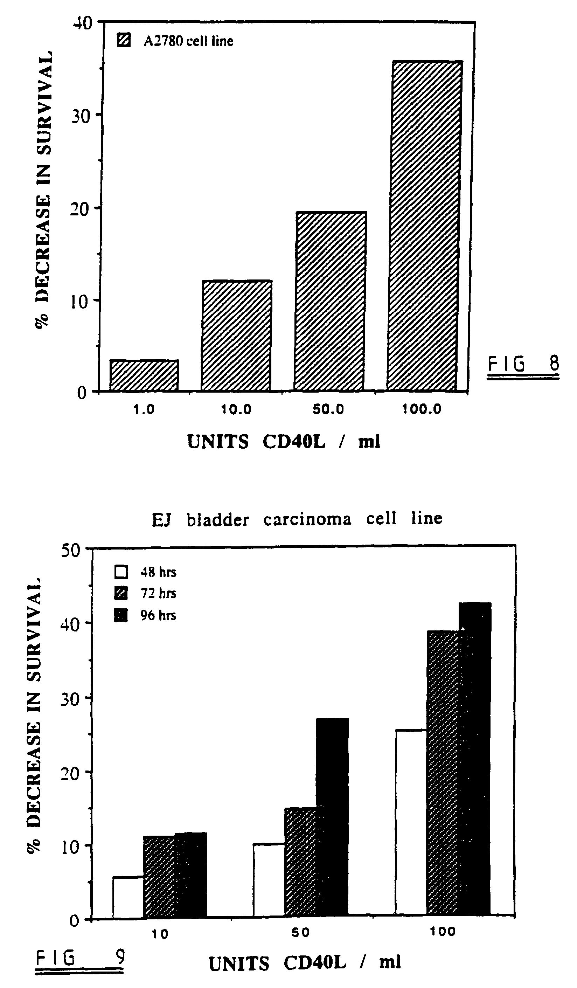

The effect of the CD40L on the cell growth of the A2780, 2780CP and EJ carcinoma cell lines as well as a CD40 Rat-1 transfectant (Rat-1/CD40 CI.8) was examined. 100 U/ml of CD40L induced a 36% decrease in survival of the ovarian A2780 cell line relative to control after 48 hrs treatment (Fig. 8). The effect of CD40L on the EJ cell line is both time and concentration-dependent with maximum effect at 96 hrs where treatment with 100 U/ml CD40L induced a 43% decrease in survival (Fig. 9).

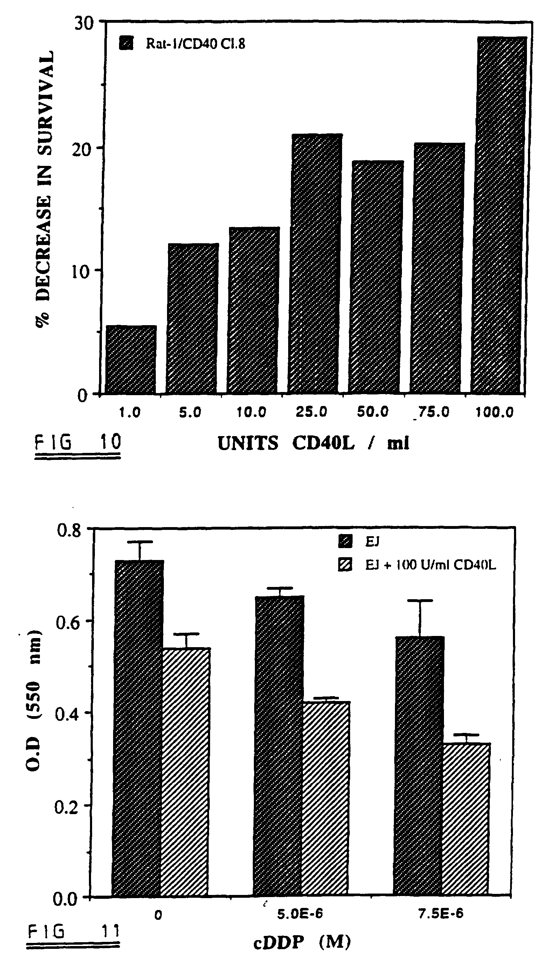

The specificity of the CD40 effect was investigated using Rat-1 fibroblasts expressing a transfected CD40. As demonstrated in Fig. 10, stimulation of CD40 by CD40L inhibited cell growth. In the transfectant studied (clone 8), incubation for 48 hrs with 100 U/ml CD40L induced a 28% decrease (Figure. 10).

### Enhancement of cis-platin-induced cell death by CD40 activation

Treatment of the EJ carcinoma cell line with 7.5 *µ*M *cis*-platin in the presence of 100 U/ml of CD40L showed enhancement of cell death (Figure 11).

## Claims

1. The use of a CD40 receptor binder for the manufacture of a medicament for the prevention of epithelial cell proliferation.

2. The use of a CD40 receptor binder for the manufacture of a medicament for the treatment of carcinomas in epithelial cell systems.

3. The use in accordance with claim 1 or claim 2 in combination with a CD40 inducer.

4. The use in accordance with claim 3, wherein said CD40 inducer is selected from the group consisting of IFN-γ and TNF-α.

5. The use of a CD40 receptor binder in the manufacture of a medicament for enhancing the susceptibility of neoplastic epithelial cells to anti-neoplastic drug-induced apoptosis.

6. The use in accordance with claim 2 or claim 5 in combination with an anti-neoplastic agent.

7. The use of a CD40 receptor binder in the manufacture of a medicament for enhancing the susceptibility of neoplastic epithelial cells which exhibit resistance to an anti-neoplastic agent, to anti-neoplastic drug-induced apoptosis, in combination with the anti-neoplastic agent.

8. The use in accordance with claim 6 or claim 7, wherein said anti-neoplastic agent is selected from the group consisting of transforming growth factor β₁, anti-Fas, tumour necrosis, factor-α, interferon-γ, cis-platin, mitomycin C, cyclophosphamide, actinomycin D, doxorubicin, vincristine, etoposide, 5-fluorouracil and methotrexate.

9. The use as claimed in any preceding claim, wherein the CD40 receptor binder is selected from ligand mimetic monoclonal antibodies and naturally occurring or engineered forms of CD40 ligands.

10. The use as claimed in any preceding claim, wherein the CD40 receptor binder is selected from G28.5 (ATCC HB-9110) and CD40L.

## Patentansprüche

1. Verwendung eines CD40 Rezeptorbindemittels für die Herstellung eines Arzneimittels für die Verhütung von Epithelzellproliferation.

2. Verwendung eines CD40 Rezeptorbindemittels für die Herstellung eines Arzneimittels für die Behandlung von Karzinomen in Epithelzellsystemen.

3. Verwendung nach Anspruch 1 oder Anspruch 2 in Kombination mit einem CD40 Inducer.

4. Verwendung nach Anspruch 3, wobei der CD40 Inducer aus der Gruppe, bestehend aus IFN-γ und TNF-α, ausgewählt ist.

5. Verwendung eines CD40 Rezeptorbindemittels bei der Herstellung eines Arzneimittels zum Erhöhen der Suszeptibilität von neoplastischen Epithelzellen gegenüber durch anti-neoplastisches Arzneimittel-induzierter Apoptosis.

6. Verwendung nach Anspruch 2 oder Anspruch 5 in Kombination mit einem anti-neoplastischen Mittel.

7. Verwendung eines CD40 Rezeptorbindemittels bei der Herstellung eines Arzneimittels zum Erhöhen der Suszeptibilität von neoplastischen Epithelzellen, die Resistenz gegenüber einem anti-neoplastischen Mittel, gegenüber durch anti-neoplastisches Arzneimittel-induzierter Apoptosis, in Kombination mit dem anti-neoplastischen Mittel zeigen.

8. Verwendung nach Anspruch 6 oder Anspruch 7, wobei das anti-neoplastische Mittel aus der Gruppe, bestehend aus Transformationswachstumsfaktor β₁, anti-Fas, Tumornekrosis Faktor-α, Interferon-γ, Cisplatin, Mitomycin C, Cyclophosphamid, Actinomycin D, Doxorubicin, Vincristin, Etoposid, 5-Fluoruracil und Methotrexat, ausgewählt ist.

9. Verwendung nach einem der vorhergehenden Ansprüche, wobei das CD40 Rezeptorbindemittel aus Ligand mimetischen monoklonalen Antikörpern und natürlich vorkommenden oder entwickelten Formen von CD40 Liganden ausgewählt ist.

10. Verwendung nach einem der vorhergehenden Ansprüche, wobei das CD40 Rezeptorbindemittel aus G28.5 (ATCC HB-9110) und CD40L ausgewählt ist.

## Revendications

1. Utilisation d'un liant du récepteur CD40 pour la fabrication d'un médicament pour la prévention de la prolifération de cellules épithéliales.

2. Utilisation d'un liant du récepteur CD40 pour la fabrication d'un médicament pour le traitement de carcinomes dans des systèmes de cellules épithéliales.

3. Utilisation selon la revendication 1 ou la revendication 2, en association avec un inducteur de CD40.

4. Utilisation selon la revendication 3, dans laquelle ledit inducteur de CD40 est choisi dans le groupe constitué par IFN-γ et TNF-α.

5. Utilisation d'un liant du récepteur CD40 dans la fabrication d'un médicament pour accroître la susceptibilité de cellules épithéliales néoplasiques à une apoptose due à un médicament antinéoplasique.

6. Utilisation selon la revendication 2 ou la revendication 5, en association avec un agent antinéoplasique.

7. Utilisation d'un liant du récepteur CD40 dans la fabrication d'un médicament pour accroître la susceptibilité de cellules épithéliales néoplasiques qui présentent de la résistance à un agent antinéoplasique, à une apoptose due à un médicament antinéoplasique, en association avec l'agent antinéoplasique.

8. Utilisation selon la revendication 6 ou la revendication 7, dans laquelle ledit agent antinéoplasique est choisi dans le groupe constitué par le facteur de croissance transformant β₁, anti-Fas, le facteur de nécrose tumorale α, l'interféron-γ, le cisplatine, la mitomycine C, le cyclophosphamide, l'actinomycine D, la doxorubicine, la vincristine, l'étoposide, le 5-fluoro-uracile et le méthotrexate.

9. Utilisation selon l'une quelconque des revendications précédentes, dans laquelle le liant du récepteur CD40 est choisi parmi des anticorps monoclonaux mimétiques d'un ligand et les formes de ligands de CD40 naturelles ou modifiées.

10. Utilisation selon l'une quelconque des revendications précédentes, dans laquelle le liant du récepteur CD40 est choisi parmi G28.5 (ATCC HB-9110) et CD40L.
